# EUROPÄISCHE PATENTANMELDUNG

(11) **EP 3 336 455 A1**
(43) Veröffentlichungstag der Anmeldung: **20.06.2018**
(21) Anmeldenummer: 17207515.2
(22) Anmeldetag: 14.12.2017
(51) Int. Cl.: F25D 3/11, F25D 25/02

(54) **KRYOLAGERUNGSVORRICHTUNG ZUR LAGERUNG VON PROBEN, INSBESONDERE ZUR TIEFTEMPERATUR-LAGERUNG VON BIOLOGISCHEN PROBEN**

(30) Priorität: 16.12.2016 DE 102016124720
(71) Anmelder: KD Maennel GmbH, 01689 Weinwöhla (DE)
(72) Erfinder: Männel, Johannes, 01689 Weinböhla (DE)
(74) Vertreter: Riechelmann & Carlsohn Patentanwälte

(57) **Zusammenfassung**

Die Erfindung betrifft eine Kryolagerungsvorrichtung zur Lagerung von Proben, insbesondere zur Tieftemperatur-Lagerung von biologischen Proben, aufweisend einen thermisch isolierten Lagerungsbehälter (2) mit einem Behälterboden (3) und einer Behälterdecke (4), die mittels Seitenwänden (5) miteinander verbunden sind, wobei in dem Lagerungsbehälter (2) zumindest zwei Lagerungseinheiten (6, 6') ausgebildet sind, zwischen denen eine Handhabungseinheit (7) angeordnet ist. Dabei ist vorgesehen, dass
- die Lagerungseinheiten (6, 6') jeweils eine vertikal verlaufende Welle (8, 8') aufweisen, an der Probenträger (9) zur Aufnahme von Probenbehältern lösbar befestigt werden können;
- die Handhabungseinheit (7) eine vertikal verlaufende Welle (10) aufweist, an der ein Schlitten (11) läuft, wobei der Schlitten (11) in vertikaler Richtung entlang der Welle (10) beweglich ist, um eine Drehachse (A) drehbar ist, die auf der Welle (10) der Handhabungseinheit liegt, und ein in horizontaler Richtung bewegliches Greifmittel (12) aufweist, das in lösbaren Eingriff mit einem Probenträger (9) gebracht werden kann; und
- zumindest in einer Seitenwand (5) des Lagerungsbehälters (2) ein verschließbarer Zugang (13) ausgebildet ist, zu dem der Probenträger (9) mittels des Schlittens (11) und des Greifmittels (12) führbar ist.

## Beschreibung

Die Erfindung betrifft eine Kryolagerungsvorrichtung zur Lagerung von Proben, insbesondere zur Tieftemperatur-Lagerung von biologischen Proben. Sie betrifft ferner ein Verfahren zur Lagerung von Proben unter Verwendung dieser Vorrichtung.

Biologische Proben, beispielsweise Knochenmark, Stammzellen, Samenzellen oder Gewebe, können lange Zeiträume konserviert werden, wenn sie bei Temperaturen weit unter dem Gefrierpunkt gelagert werden. Insbesondere eine Lagerung bei Temperaturen von -140 °C oder weniger, der sogenannten Tieftemperatur-Lagerung, sorgt für eine wirksame und dauerhafte Konservierung der Proben, auch über Zeiträume von mehreren Jahrzehnten. Die technischen Anforderungen an die für eine Tieftemperatur-Lagerung benötigten Vorrichtungen sind jedoch hoch. Die Vorrichtungen, die in der Regel eine große Zahl an Proben aufnehmen sollen, müssen für eine Aufrechterhaltung der Kühlkette sorgen, auch wenn Proben aus der Vorrichtung entnommen oder in die Vorrichtung eingebracht werden. Die Ein- und Auslagerung von Proben in diese Vorrichtungen erfordert somit spezielle Handhabungseinrichtungen, die es erlauben, Proben in einen gekühlten Lagerbehälter einzuführen oder daraus zu entnehmen, ohne die Kühlung der bereits in dem Lagerbehälter befindlichen Proben zu beeinträchtigen.

Typischerweise werden die Proben zur Konservierung in Probengefäße, beispielsweise Vials, Goblets, Straws, Nabelschnurblutkassetten, eingebracht. Die Probengefäße werden dann auf Probenträger, die auch als Trays bezeichnet werden, an einer vorgegebenen Position gegeben. Das Tray kann dazu ein Aufnahmeraster aufweisen, das Spalten (X-Achse) und Reihen (Y-Achse) vorsieht. Die Trays wiederum können eine Kennung tragen, beispielsweise einen Strichcode. Die Trays werden in einigen Fällen in Trägergestelle, sogenannte Racks, eingeführt. Ein Rack kann mehrere Trays aufnehmen, die typischerweise übereinander in dem Rack angeordnet sind. In einen Lagerbehälter können in der Regel mehrere Racks nebeneinander eingebracht sein. Der Lagerbehälter ist zumeist ein supervakuumisolierter Behälter. Dazu kann der Behälter einen Doppelmantel aus Edelstahl aufweisen, wobei zwischen den beiden Mänteln des Doppelmantels Vakuum ausgebildet ist. Als Kältemittel wird vorwiegend flüssiger Stickstoff eingesetzt, so dass in dem Lagerbehälter Temperaturen von -196 °C durch Verdampfen des flüssigen Stickstoffes erreicht werden können.

Bekannte Vorrichtungen zur Tieftemperaturlagerung biologischer Proben sehen in der Regel eine automatisierte Ein- und Auslagerung von Proben in den Lagerbehälter vor. Eine manuelle Handhabung der Proben wird damit vermieden. Eine manuelle Handhabung ist mit hohen Risiken verbunden, weil in dem Lagerbehälter enthaltene Proben höheren Temperaturen ausgesetzt werden können und die Gefahr von Verwechslungen, die aus einer falschen Zuordnung der Proben und ihrer Lagerplätze resultiert, besteht. Die Höhe der Lagerbehälter ist begrenzt, weil die manuelle Handhabung der Proben die Zugänglichkeit der Proben für einen Anlagenbediener erfordert, was nur über eine bestimmte Behälterhöhe möglich ist. Schließlich muss oberhalb des Lagerraums ein genügend großer Raum vorgesehen sein, um das Rack, in dem sich das Tray mit dem die Probe enthaltenden Probengefäß befindet, nach oben aus dem Lagerbehälter führen zu können.

Bei Vorrichtungen mit einer automatisierten Ein- und Auslagerung von Proben ist zumeist eine gesonderte Überführungskammer vorgesehen, die oberhalb des Lagerbehälters angeordnet und in der Regel gekühlt ist. DE 10 2004 053 170 B4 sieht eine gekühlte Aufnahme- und Abgabekammer vor, die oberhalb der Lagerungskammer angeordnet ist. Mittels einer manuell angetriebenen Mechanik können Probenbehälter aus der Aufnahme- und Abgabekammer in die Lagerungskammer überführt werden. Dort werden sie in Trays gelagert, die an einer vertikalen Welle befestigt sind. EP 1 543 278 B1 und DE 10 2011 012 887 B4 erfordern Aufnahme- und Abgabekammern, deren Größe nicht nur durch die Dimensionen des Probenbehälters, sondern darüber hinaus durch Komponenten der Einrichtungen zum Transfer von Probenbehältern zwischen der Lagerungskammer und der Aufnahme- und Abgabekammer bestimmt wird. In beiden Fällen ist die Höhe der Aufnahme- und Abgabekammer gleich der Höhe der Lagerungskammern. Eine weitere Variante dieser Bauform wird in US 9,151,770 B2 beschrieben. Dort muss das Rack vom Boden der Lagerungskammer in eine Transferkammer angehoben werden, um einen Probenbehälter aus der Lagerungskammer entnehmen zu können.

Die Höhe von Räumen, in denen die Lagerungsvorrichtungen für die Tieftemperatur-Lagerung von biologischen Proben aufgestellt werden, ist jedoch begrenzt. Die nach dem Stand der Technik erforderliche Höhe der Aufnahme- und Abgabekammern oder Transferkammern beschränkt damit die Höhe der Lagerungskammern. Damit wird die Nutzbarkeit des Raumes für die Aufstellung von Lagerungsvorrichtungen begrenzt. Darüber hinaus erfordert der Transfer der Probenbehälter aus der Lagerungskammer in die Aufnahme- und Abgabekammern und unter Umständen von dort, wie in DE 10 2011 012 887 B4 beschrieben, in einen Arbeitsraum eine Kühlung der Aufnahme- und Abgabekammern. Eine solche Kühlung ist entweder technisch und energetisch aufwendig oder aber unzureichend, so dass die Proben dann nicht mehr der erforderlichen Kühlung ausgesetzt sind.

Aufgabe der Erfindung ist es, die Nachteile nach dem Stand der Technik zu beseitigen. Es soll insbesondere eine Vorrichtung zur Lagerung von Proben, insbesondere zur Tieftemperatur-Lagerung von biologischen Proben, angegeben werden, die eine bessere Nutzung der Raumhöhe erlaubt. Außerdem soll die Kühlung der Proben bei einem Transfer besser gewährleistet werden. Ferner soll ein Verfahren zur Lagerung von Proben mittels einer solchen Vorrichtung angegeben werden.

Aus EP 2 354 729 A1 ist eine Vorrichtung zur Einstellung tiefkalter Temperaturen bekannt. Die Vorrichtung weist eine Kältekammer mit einem ersten Zugang und eine Schleusenkammer mit einem zweiten Zugang auf. Der erste Zugang ist in einer Seitenwand der Kältekammer ausgebildet. Der zweite Zugang befindet sich allerdings an der Oberseite der Schleusenkammer. Aus US 4,236,518 B1 ist eine kryogene Vorrichtung bekannt, die ein unter Druck stehendes Kühlgas verwendet.

Diese Aufgabe wird durch die Merkmale der Ansprüche 1 und 14 gelöst. Zweckmäßige Ausgestaltungen der Erfindungen ergeben sich aus den Merkmalen der Unteransprüche.

Nach Maßgabe der Erfindung ist eine Kryolagerungsvorrichtung zur Lagerung von Proben, insbesondere zur Tieftemperatur-Lagerung von biologischen Proben, vorgesehen, die einen thermisch isolierten Lagerungsbehälter mit einem Behälterboden und einer Behälterdecke, die mittels Seitenwänden miteinander verbunden sind, aufweist. Dabei sind in dem Lagerungsbehälter zumindest zwei Lagerungseinheiten ausgebildet, zwischen denen eine Handhabungseinheit angeordnet ist, wobei
- die Lagerungseinheiten jeweils eine vertikal verlaufende Welle aufweisen, an der Probenträger zur Aufnahme von Probenbehältern lösbar befestigt werden können;
- die Handhabungseinheit eine vertikal verlaufende Welle aufweist, an der ein Schlitten läuft, wobei der Schlitten in vertikaler Richtung entlang der Welle beweglich ist, um eine Drehachse drehbar ist, die auf der Welle der Handhabungseinheit liegt, und ein in horizontaler Richtung bewegliches Greifmittel aufweist, das in lösbaren Eingriff mit einem Probenträger gebracht werden kann; und
- zumindest in einer Seitenwand des Lagerungsbehälters ein verschließbarer Zugang ausgebildet ist, zu dem der Probenträger mittels des Schlittens und des Greifmittels führbar ist.

Die erfindungsgemäße Vorrichtung ermöglicht den Transport eines Probenträgers zwischen einer Übergabeposition und einer Lagerungsposition. Dabei ist der Probenträger in der Übergabeposition, wenn er sich an dem verschließbaren Zugang, der in einer Seitenwand des Lagerungsbehälters ausgebildet ist, befindet. Der Probenträger ist hingegen in seiner Lagerungsposition, wenn er sich in einer vorgegebenen Lagerungseinheit an einer vorgegebenen Position an einer Welle der Lagerungseinheit befindet. Die für diesen Transport erforderliche Handhabungseinheit befindet sich, abgesehen von einem Teil der Welle und des erforderlichen Antriebs für die Welle, innerhalb des Lagerungsbehälters. Die für den Transport erforderliche Mechanik der Handhabungseinheit, insbesondere der Schlitten und das Greifmittel, sind somit denselben klimatischen Bedingungen ausgesetzt, wie die Lagerungseinheiten und die dort gelagerten Probenträger. Das gilt insbesondere für die in dem Lagerungsbehälter vorherrschende Temperatur. Dabei liegt die Lagerungstemperatur vorzugsweise bei Temperaturen unter 0 °C, besonders bevorzugt bei Temperaturen zwischen -196 °C und -150 °C.

Die erfindungsgemäße Vorrichtung zeichnet sich insbesondere dadurch aus, dass kein zusätzlicher Raum oberhalb des Lagerungsbehälters benötigt wird, um die Probenträger aus dem Lagerungsbehälter zu entnehmen. Die Probenträger werden nicht über die Behälterdecke hinaus befördert. Eine Bewegung der Probenträger in vertikaler Richtung findet nur innerhalb des Lagerungsbehälters statt. Die Lagerungseinheiten insgesamt können und müssen nicht angehoben werden, um einen Probenträger aus einer Lagerungseinheit zu entnehmen. Oberhalb der Behälterdecke sind Lager, Getriebe und/oder Antriebe angeordnet, die benötigt werden, um die erforderlichen Bewegungen der Lagerungseinheiten und der Handhabungseinheit sowie gegebenenfalls von Komponenten dieser Einheiten bewirken zu können.

Die erfindungsgemäße Vorrichtung benötigt somit eine wesentlich geringere Raumhöhe als die bisher bekannten Vorrichtungen des Standes der Technik. Darüber hinaus sorgt die Handhabung der Probenträger innerhalb des Lagerungsbehälters dafür, dass die anderen Probenträger den Lagerungsbehälter nicht, auch nicht kurzzeitig, verlassen, nur um einen Probenträger aus dem Lagerungsbehälter entnehmen zu können. Damit beeinträchtigt die Entnahme oder das Einführen eines Probenträgers in den Lagerungsbehälter nicht die Integrität der Probenbehälter, die sich bereits in dem Lagerungsbehälter befinden.

Die erfindungsgemäße Kryolagerungsvorrichtung kann mehr als zwei Lagerungseinheiten aufweisen. Dabei sollte jede der Lagerungseinheiten an die Handhabungseinheit angrenzen. Die Handhabungseinheit sollte dabei an einer Seitenwand angeordnet sein, d. h., dass zumindest an einer Seitenwand des Lagerungsbehälters keine Lagerungseinheit zwischen der Seitenwand und der Handhabungseinheit angeordnet ist.

Es kann vorgesehen sein, dass die Wellen der Lagerungseinheiten und die Welle der Handhabungseinheit durch die Behälterdecke geführt sind, wobei jede der Wellen in einer Lagereinheit außerhalb des Lagerungsbehälters gelagert ist. Eine Lagerung der Welle in einem Lager, das sich in dem Lagerungsbehälter befindet, ist nicht erforderlich. Zumindest bei einer der Wellen, vorzugsweise bei allen Wellen, kann vorgesehen sein, dass das Ende der Welle, das dem Behälterboden zugewandt ist, vom Behälterboden beabstandet ist. Damit verbleibt zwischen dem Behälterboden und den Wellen der Lagerungseinheiten ein Raum. Dieser Raum kann beispielsweise zur Aufnahme von flüssigem Stickstoff oder einem anderen Kältemittel dienen. Auf diese Weise wird sichergestellt, dass keine Lagerkomponenten für die Welle im tiefkalten Bereich angeordnet sind. Die außerhalb des Lagerungsbehälters angeordneten Lagereinheiten sind hingegen leicht zugänglich, was eine Wartung der Lagereinheiten erleichtert. Es kann daher vorgesehen sein, dass sich eine oder mehrere, vorzugsweise alle Wellen innerhalb des Lagerungsbehälters in Richtung des Behälterbodens erstrecken, ohne in einer Lagereinheit am Behälterboden und/oder einer anderen Lagereinheit gelagert zu sein.

Vorzugsweise sind außerhalb des Lagerungsbehälters Antriebe für die Wellen der Lagerungseinheiten angeordnet. Ferner kann außerhalb des Lagerungsbehälters ein Antrieb für die Welle der Handhabungseinheit angeordnet sein. Die Antriebe können dazu oberhalb der Behälterdecke des Lagerungsbehälters angeordnet sein. Die Antriebe für die Wellen der Lagerungseinheiten sollen eine Drehung der Wellen um deren Längsachse bewirken. Dabei kann vorgesehen sein, dass eine Drehung nur um bestimmte Winkel möglich ist. Beispielsweise kann vorgesehen sein, dass eine Drehung nur um einen Winkel von 60° oder ganzzahligen Vielfachen davon möglich ist. Damit kann die Welle einer Lagerungseinheit nur bestimmte Positionen erreichen.

An der Welle einer Lagerungseinheit können die Probenträger in mehreren, in vertikaler Richtung übereinander liegenden Ebenen befestigbar sein. Die Ebenen einer Lagerungseinheit sollten äquidistant voneinander ausgebildet sein. In einer Ebene können mehrere Probenträger an der Welle befestigbar sein. Dazu kann jede der Ebenen in gleiche Teile gegliedert sein, wobei jeder Teil der Ebene einen Probenträger aufnehmen kann. Die Teile einer Ebene werden im Folgenden auch als Lagerungssektoren bezeichnet. Sind alle Teile einer Ebene mit Probenträgern belegt, so bilden die Probenträger gemeinsam eine ringförmige Anordnung um die Welle herum. Die Probenträger weisen zweckmäßigerweise einen der Welle zugewandten, inneren Rand und einen der Welle abgewandten, äußeren Rand auf. Der innere Rand und der äußere Rand eines Probenträgers sind durch radial verlaufende Seitenränder verbunden. Der innere Rand ist schmaler als der äußere Rand. In einer Ebene liegende benachbarte Probenträger sind einander jeweils mit einem ihrer Seitenränder zugewandt. Die einander zugewandten Seitenränder zweier benachbarter Probenträger können aneinander anliegen oder voneinander unter Ausbildung eines radial zur Welle verlaufenden Spaltes voneinander beabstandet sein. Die Zahl der Teile der Ebene entspricht dabei der Zahl der Positionen, die die Welle durch ihre Drehung erreichen kann. Ist beispielsweise eine Drehung der Welle um 60° oder ganzzahlige Vielfache davon vorgesehen, so weist eine Ebene sechs Teile bzw. Lagerungssektoren auf. Das heißt mit anderen Worten, dass die Zahl der Probenträger, die sich in einer Ebene befinden können, der Zahl der Teile einer Ebene entspricht, der wiederum die Zahl der Positionen entspricht, in die die Welle der Lagerungseinheit gedreht werden kann. Zweckmäßigerweise kann jede Ebene dieselbe Zahl von Probenträgern aufnehmen.

Die äußeren Ränder der Probenträger können die Außenkanten der Probenträger bilden. Dabei können die Außenkanten der Probenträger, die in einer Ebene angeordnet sind, entlang eines Kreises oder Kreisbogens um die Welle der Lagerungseinheit angeordnet sein.

Zur lösbaren Befestigung eines Probenträgers an einer Welle können an der Welle Halteelemente ausgebildet sein. Die Halteelemente sind an der Welle so angeordnet, dass die Zahl der Halteelemente in einer Ebene der Zahl der Teile der Ebene entspricht. Dabei ist in jeder Ebene die Zahl von Halteelementen vorgesehen, die der Zahl der Teile einer Ebene entspricht. Für jeden Probenträger können dabei ein oder mehrere Halteelemente vorgesehen sein. Das oder die Halteelemente, die zur lösbaren Befestigung eines Probenträgers vorgesehen sind, sind zweckmäßigerweise äquidistant um die Welle angeordnet.

Es kann vorgesehen sein, dass die Halteelemente in korrespondierende Aufnahmen eingreifen, die an den Probenträgern ausgebildet sind. Zweckmäßigerweise sind diese Aufnahmen an dem inneren Rand der Probenträger ausgebildet. Die Halteelemente können hakenartige Elemente sein, in die Probenträger über ihre Aufnahmen, beispielsweise Öffnungen, eingehängt werden können.

Erfindungsgemäß weist die Handhabungseinheit eine vertikal verlaufende Welle auf, an der ein Schlitten läuft. Der Schlitten ist in vertikaler Richtung entlang der Welle der Handhabungseinheit beweglich und um eine Drehachse drehbar, die auf der Welle der Handhabungseinheit liegt. Der Schlitten weist ein in horizontaler Richtung bewegliches Greifmittel auf, das in Eingriff mit einem Probenträger gebracht werden kann. Dazu können an den äußeren Rändern der Probenträger Ausnehmungen ausgebildet sein, in die das Greifmittel eingreifen kann.

Mittels des Greifmittels können Probenträger an den Wellen der Lagerungseinheiten befestigt werden. Dazu wird zunächst der Teil einer Ebene einer Lagerungseinheit vorgegeben, an dem der Probenträger in dem Lagerungsbehälter gelagert werden soll. Dieser Teil wird im Folgenden auch als Lagerungssektor bezeichnet. Der Lagerungssektor für einen Probenträger ist durch die Lagerungseinheit, in der sich der Lagerungssektor befindet, die Ebene, in der sich der Lagerungssektor befindet, und den Teil der Ebene gekennzeichnet, zu dem der Probenträger geführt werden und den er einnehmen soll. Um einen Probenträger zu dem vorgegebenen Lagerungssektor zu führen und dort an der Welle der Lagerungseinheit zu befestigen, wird die Lagerungseinheit mittels ihrer Welle gedreht, bis der vorgegebene Lagerungssektor der Handhabungseinheit zugewandt ist. Anschließend oder parallel dazu wird der Schlitten, dessen Greifmittel den Probenträger hält, der zu dem vorgegebenen Lagerungssektor geführt werden soll, in Höhe der Ebene geführt, in der sich der Lagerungssektor befindet. Der Schlitten wird so ausgerichtet, dass der vom Greifmittel gehaltene Probenträger mit seinem inneren Rand der Lagerungseinheit, in der sich der Lagerungssektor befindet, zugewandt ist. Das kann durch ein Drehen des Schlittens um die Welle erfolgen. Das Greifmittel wird nun horizontal in Richtung der Lagerungseinheit bewegt. Sobald die Aufnahmen, die am inneren Rand des Probenträgers ausgebildet sind, in die korrespondierenden Halteelemente der Welle der Lagerungseinheit eingreifen, so dass der Probenträger an der Welle befestigt ist, wird die Verbindung zwischen dem Greifmittel und dem Probenträger gelöst, beispielsweise indem das Greifmittel durch eine Bewegung des Schlittens leicht abgesenkt wird. Sodann wird das Greifmittel zurück in Richtung der Welle der Handhabungseinheit geführt. Der Schlitten der Handhabungseinheit kann dann in eine andere Ebene geführt werden oder mittels seines Greifmittels - ohne Höhenveränderung - einen anderen oder denselben Probenträger aus einer Ebene einer Lagerungseinheit aufnehmen, in dessen Höhe er sich bereits befindet.

Die Drehung des Schlittens um die Welle der Handhabungseinheit kann beispielsweise durch eine Drehung der Welle bewirkt werden. Zur Höhenverstellung des Schlittens kann ein Antriebsgestänge vorgesehen sein, das über ein Getriebe mittels eines Antriebs angetrieben wird, der außerhalb des Lagerungsbehälters, beispielsweise an der Behälterdecke angeordnet ist. Zur Bewegung des Greifmittels kann ein Motor vorgesehen sein, beispielsweise ein Servomotor. Der Motor kann über ein Schubgestänge die horizontale Bewegung des Greifmittels bewirken. Der Servomotor ist vorzugsweise außerhalb des Lagerungsbehälters, beispielsweise an der Behälterdecke angeordnet. Er kann aber auch im Behälter, beispielsweise am Schlitten, angeordnet sein.

Zur Entnahme eines Probenträgers aus seinem Lagerungssektor wird die Lagerungseinheit, in der sich der Lagerungssektor mit dem Probenträger befindet, mittels ihrer Welle gedreht, bis der Lagerungssektor der Handhabungseinheit zugewandt ist. Der Schlitten wird, sofern er sich nicht bereits in dieser Ebene befindet, in Höhe der Ebene geführt, in der sich der Lagerungssektor mit dem Probenträger befindet. Außerdem wird der Schlitten so ausgerichtet, dass er dem Lagerungssektor, in dem sich der Probenträger befindet, zugewandt ist. Anschließend wird das Greifmittel horizontal in Richtung des Probenträgers geführt, bis es in die Ausnehmungen, die am äußeren Rand des Probenträgers ausgebildet sind, eingreifen kann. Sobald die Verbindung zwischen dem Greifmittel und dem Probenträger hergestellt ist, wird die Verbindung zwischen dem inneren Rand des Probenträgers und den Halteelementen an der Welle gelöst, beispielsweise indem das Greifmittel durch eine Bewegung des Schlittens leicht angehoben wird. Sodann wird das Greifmittel mit dem Probenträger zurück in Richtung der Welle der Handhabungseinheit geführt. Der Schlitten der Handhabungseinheit kann dann in eine andere Ebene geführt werden oder mittels seines Greifmittels - ohne Höhenveränderung - den Probenträger zu einem anderen oder demselben Lagerungssektor in einer Ebene einer Lagerungseinheit führen, in dessen Höhe er sich bereits befindet.

Der vertikale Abstand der Ebenen wird durch die Höhe der Probenbehälter bestimmt. Dabei sollte der Abstand zwischen den Ebenen etwas größer als die Höhe der Probenbehälter sein. Auf diese Weise können die Probenträger beispielsweise abgesenkt werden, wenn die Probenträger an einer Welle befestigt werden, indem der Probenträger in die Halteelemente eingehängt wird. Zur Entnahme des Probenträgers von der Welle kann dann der Probenträger angehoben werden, bis die Halteelemente aus den Aufnahmen gelöst werden können. Anschließend kann der Probenträger in horizontaler Richtung von der Welle weg bewegt werden.

Zweckmäßigerweise weisen alle Probenträger die gleichen Dimensionen auf. An den Unterkanten der Probenträger ist ein Boden ausgebildet, der sich vom inneren Rand zum äußeren Rand und von dem einen Seitenrand zu dem anderen Seitenrand erstreckt. Der Boden kann Öffnungen aufweisen, beispielsweise Öffnungen, in die korrespondierende Elemente, die an den Unterseiten der Probenbehälter ausgebildet sind, eingreifen können.

Zur Einstellung der Lagerungstemperatur in dem Lagerungsbehälter kann ein Kältemittel, beispielsweise flüssiger Stickstoff, eingesetzt werden. Alternativ oder zusätzlich kann die Kälte auch elektrisch erzeugt werden.

Innerhalb des Lagerungsbehälters kann am Behälterboden eine Bodenkammer zur Aufnahme von flüssigem Stickstoff oder eines anderen Kältemittels ausgebildet sein. An der Behälterdecke kann zumindest eine Deckenkammer zur Aufnahme von flüssigem Stickstoff oder eines anderen Kältemittels ausgebildet sein. Dabei kann eine der Deckenkammern über eine Leitung mit der Bodenkammer verbunden sein. Im Folgenden wird die Verwendung von flüssigem Stickstoff als Kältemittel beschrieben, auch wenn anstelle des flüssigen Stickstoffs ein anderes Kältemittel in gleicher Weise eingesetzt werden kann.

Zur Erzeugung der Tiefkälte kann flüssiger Stickstoff in dem Lagerungsbehälter verdampft werden. Der flüssige Stickstoff kann drucklos verdampft werden. Dazu kann flüssiger Stickstoff, dessen Temperatur ca. -192 °C beträgt, in den Lagerungsbehälter geführt werden. Der im Lagerungsbehälter verdampfende Stickstoff sorgt für eine Kühlung der Probenbehälter, die sich in dem Lagerungsbehälter befinden. Es kann vorgesehen sein, dass die erfindungsgemäße Kryolagerungsvorrichtung Einrichtungen zur Führung von flüssigem Stickstoff in den Lagerungsbehälter aufweist. Dabei kann der flüssige Stickstoff aus einem gesonderten Versorgungsbehälter über eine Versorgungsleitung in den Lagerungsbehälter geführt werden. Zum Öffnen und Schließen der Versorgungsleitung kann zumindest ein Ventil vorgesehen sein, beispielsweise eine Kombination aus zwei Magnetventilen und einem Sicherheitsventil.

Es kann vorgesehen sein, dass der flüssige Stickstoff aus dem Versorgungsbehälter zunächst in eine oder beide Deckenkammern geführt wird. Sobald der flüssige Stickstoff in der oder den Deckenkammern eine vorgegebene Füllhöhe erreicht, kann er über die jeweilige Leitung, die die Deckenkammern mit der Bodenkammer verbindet, in die Bodenkammer geführt werden. Dazu kann vorgesehen sein, dass eine oder beide Deckenkammern einen Überlauf aufweisen. Sobald die vorgegebene Füllhöhe in der Deckenkammer erreicht ist, tritt flüssiger Stickstoff über den Überlauf in die Leitung zur Bodenkammer ein, wodurch der flüssige Stickstoff in die Bodenkammer gelangt. Es können Messgeräte zur Bestimmung der Füllstandshöhe in der Bodenkammer und/oder der oder den Deckenkammern vorgesehen sein. Sobald der Füllstand in der Bodenkammer eine vorgegebene Füllhöhe erreicht, wird die Zufuhr von Stickstoff durch Schließen der Versorgungsleitung automatisch gestoppt. Ebenso kann vorgesehen sein, dass die Zufuhr von Stickstoff durch Schließen der Versorgungsleitung automatisch gestoppt wird, wenn der flüssige Stickstoff in der oder den Deckenkammern eine vorgegebene Füllhöhe übersteigt. Auf diese Weise kann sichergestellt werden, dass kein flüssiger Stickstoff die Probenbehälter kontaktiert. Fällt hingegen die Füllhöhe des Stickstoffes in der Bodenkammer und/oder der oder den Deckenkammern unter einen vorgegebenen Wert, so kann vorgesehen sein, dass automatisch die Zufuhr von Stickstoff über die Versorgungsleitung aufgenommen wird. Damit wird sichergestellt, dass jederzeit ausreichend Kältemittel in der erfindungsgemäßen Kryolagerungsvorrichtung vorhanden ist. Zur automatisierten Befüllung des Lagerungsbehälters mit flüssigem Stickstoff können das oder die Magnetventile auf Grundlage der Signale, die von den Messgeräten empfangen werden, geöffnet oder geschlossen werden. Es kann jedoch aus Sicherheitsgründen eine Umgehungsleitung mit einem Handventil vorgesehen sein. Die Umgehungsleitung umgeht das oder die Magnetventile, die automatisch geöffnet oder geschlossen werden. Durch Öffnen des Handventils kann eine Versorgung des Lagerungsbehälters mit flüssigem Stickstoff im Notfall auch dann sichergestellt werden, wenn die automatische Befüllung über die Magnetventile versagt.

Es kann vorgesehen sein, dass der Lagerungsbehälter Lagerungskammern aufweist, in denen jeweils eine Lagerungseinheit angeordnet ist, wobei die Lagerungskammern in Richtung der Handhabungseinheit geöffnet sind. Dabei kann der Mantel einer Lagerungskammer von einer Seitenwand des Lagerungsbehälters gebildet sein und der Behälterboden die Lagerungskammer nach unten begrenzen. Der Behälterboden kann im Bereich der Lagerungskammer nach außen gewölbt sein. Eine solche Gestaltung der Lagerungseinheiten sorgt dafür, dass die Lagerungseinheiten, abgesehen von ihrer Öffnung in Richtung der Handhabungseinheit, einen kreisförmigen Querschnitt aufweisen. Das ist im Hinblick auf eine Vakuumisolierung des Lagerungsbehälters vorteilhaft. Die thermische Isolierung des Lagerungsbehälters kann erreicht werden, indem die Wandungen des Lagerungsbehälters als doppelwandige Wandungen ausgeführt werden, wobei die beiden Wandungen voneinander unter Ausbildung eines Zwischenraums beabstandet sind. In diesem Zwischenraum herrscht Unterdruck (Vakuum). Die Druckdifferenz zwischen dem Zwischenraum und der umgebenden Atmosphäre kann beispielsweise 1 bar betragen, was einem Druck von 10 t/m² Wandungsfläche entspricht. Zur Herstellung des Unterdruckes kann der Zwischenraum evakuiert werden. Zur Unterbindung des Wärmetransportes mittels Strahlung können als Strahlenschutz eine oder mehrere isolierende Materialien, beispielsweise eine Superisolation (engl. auch als "multilayer insulation" (MLI) bezeichnet), eine geschichtete Verbundisolation (engl. als "layered composite insulation" (LCI) bezeichnet), Perlit, Aerogel oder andere, eingebracht werden.

Der - abgesehen von der Öffnung zur Handhabungseinheit hin - ringförmige Querschnitt der Lagerungseinheiten verringert ungleichmäßige Belastungen der Wandungen des Lagerungsbehälters.

Es kann vorgesehen sein, dass die Seitenwand des Lagerungsbehälters, in der der verschließbare Zugang ausgebildet ist, eine als Verbindungswand dienende Seitenwand des Lagerungsbehälters ist, die zwischen den Seitenwänden zweier benachbarter Lagerungskammern ausgebildet ist. Die Verbindungswand muss nicht gekrümmt sein. Es kann jedoch vorgesehen sein, dass die Verbindungswand nach außen gewölbt ist.

Der verschließbare Zugang kann eine Schleuse sein. Über die Schleuse kann die Kryolagerungsvorrichtung mit einer Einbringungsvorrichtung verbunden sein, die einen Arbeitsraum aufweist, über den ein Probenträger von der Einbringungsvorrichtung an die Handhabungseinheit der Kryolagerungsvorrichtung oder von der Handhabungseinheit der Kryolagerungsvorrichtung an die Einbringungsvorrichtung überführbar ist. Die Einbringungsvorrichtung sollte ebenfalls wärmeisoliert sein.

Die erfindungsgemäße Vorrichtung kann eine Steuerungseinrichtung zur Steuerung der Lagerungseinheiten und der Handhabungseinheit aufweisen. Die Steuerungseinrichtung kann zur Steuerung weiterer Komponenten der erfindungsgemäßen Vorrichtung eingesetzt werden. Die erfindungsgemäße Vorrichtung kann ferner eine Datenverarbeitungseinheit aufweisen. In der Datenverarbeitungseinheit kann gespeichert sein, in welchem Lagerungssektor sich ein bestimmter Probenträger befindet und welche Lagerungssektoren frei sind. Die Datenverarbeitungseinheit kann mit der Steuerungseinrichtung über eine Datenverbindung verbunden sein. Mittels der Datenverarbeitungseinheit kann automatisch vergeben werden, zu welchem Lagerungssektor ein Probenträger gebracht werden soll. Ferner kann mittels der Datenverarbeitungseinheit automatisch bestimmt werden, aus welchem Lagerungssektor ein bestimmter Probenträger entnommen werden soll.

Nach Maßgabe der Erfindung ist ferner ein Verfahren zur Lagerung von Proben, insbesondere zur Tieftemperatur-Lagerung von biologischen Proben, mittels der erfindungsgemäßen Kryolagerungsvorrichtung vorgesehen. Dabei wird ein Probenträger mittels der Handhabungseinheit zwischen einer Übergabeposition und einer Lagerungsposition bewegt, wobei
- sich der Probenträger in der Übergabeposition an dem verschließbaren Zugang, der in einer Seitenwand des Lagerungsbehälters ausgebildet ist, befindet und
- sich der Probenträger in der Lagerungsposition in einer vorgegebenen Lagerungseinheit an einer vorgegebenen Position an einer Welle der Lagerungseinheit befindet.

Bei der vorgegebenen Position kann es sich um einen Lagerungssektor handeln, der in einer der Lagerungsebenen einer der Lagerungseinheiten des Lagerungsbehälters ausgebildet ist.

Das erfindungsgemäße Verfahren ermöglicht es, Probenträger in den Lagerungsbehälter einzuführen oder aus diesem zu entnehmen, ohne den Probenträger oder ein den Probenträger enthaltendes Rack durch eine Öffnung in der Behälterdecke führen zu müssen. Damit ist es möglich, die Raumhöhe von Räumen, in denen die erfindungsgemäßen Lagerungsbehälter aufgestellt werden, besser auszunutzen.

Weitere Einzelheiten des erfindungsgemäßen Verfahrens sind vorstehend im Zusammenhang mit der erfindungsgemäßen Vorrichtung bereits beschrieben worden.

Die Erfindung wird nachstehend anhand von Ausführungsbeispielen, die die Erfindung nicht einschränken sollen, unter Bezugnahme auf die Zeichnungen näher erläutert. Dabei zeigen
- Fig. 1: eine schematische Schnittdarstellung einer ersten Ausführungsform einer erfindungsgemäßen Kryolagerungsvorrichtung entlang einer vertikalen Schnittebene;
- Fig. 2: eine schematische Seitenansicht der in Fig. 1 gezeigten Ausführungsform;
- Fig. 3: eine schematische Ansicht der in Fig. 1 gezeigten Ausführungsform von oben;
- Fig. 4: eine schematische Schnittdarstellung der in Fig. 1 gezeigten Ausführungs-form entlang einer horizontalen Schnittebene;
- Fig. 5: eine schematische Ansicht einer zweiten Ausführungsform von oben; und
- Fig. 6: eine Ausführungsform eines Probenträgers.

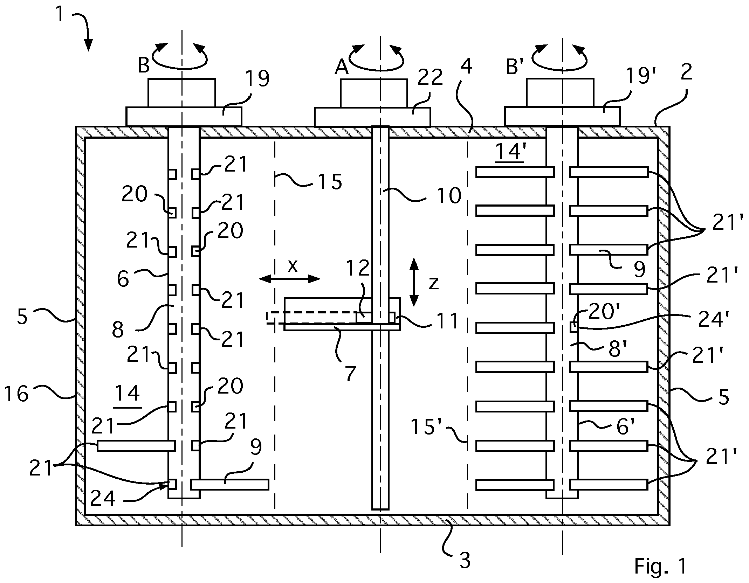

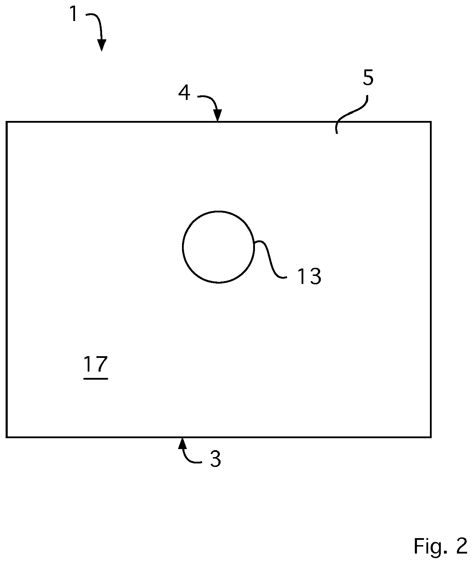

Die in den Figuren 1 bis 4 gezeigte Ausführungsform der erfindungsgemäßen Kryolagerungsvorrichtung 1 weist einen Lagerungsbehälter 2 auf, der einen Behälterboden 3 und eine Behälterdecke 4 besitzt. Der Behälterboden 3 und die Behälterdecke 4 sind über Seitenwände 5 miteinander verbunden. In dem Lagerungsbehälter 2 sind zwei Lagerungseinheiten 6, 6' und eine Handhabungseinheit 7 angeordnet. Elemente der zweiten Lagerungseinheit 6' weisen dabei das Bezugszeichen der ersten Lagerungseinheit, ergänzt um einen Oberstrich, auf. Die Seitenwände 5 des Lagerungsbehälters 2 sind so ausgebildet, dass innerhalb des Lagerungsbehälters 2 zwei Lagerungskammern 14, 14' ausgebildet sind. Die Lagerungskammern 14, 14' sind zur Handhabungseinheit 7 hin über Öffnungen 15, 15' offen, die sich in dem Lagerungsbehälter 2 von dem Behälterboden 3 bis zur Behälterdecke 4 erstrecken. Die Abschnitte 16, 16' der Seitenwände 5 des Lagerungsbehälters 2 umschließen, abgesehen von den Öffnungen 15, 15', die Lagerungskammern 14, 14', so dass die Lagerungskammern einen annähernd kreisförmigen Querschnitt aufweisen. Die Abschnitte 16, 16' der Seitenwände 5 sind über Verbindungswände 17, 18, die ebenfalls Seitenwände 5 des Lagerungsbehälters 2 sind, miteinander verbunden. Zwischen den Verbindungswänden 17, 18 ist die Handhabungseinheit 7 angeordnet. In der Verbindungswand 17 ist ein Zugang 13 zum Lagerungsbehälter 2 ausgebildet. Die Abschnitte 16, 16' der Seitenwände 5 des Lagerungsbehälters 2 entsprechen den Mänteln der Lagerungskammern 14, 14'.

Die Lagerungseinheiten 6, 6' weisen jeweils eine vertikal verlaufende Welle 8, 8' auf, die durch die Behälterdecke 4 in den Lagerungsbehälter 2 geführt sind und sich in Richtung des Behälterbodens 3 erstrecken. Dabei sind die Enden der Wellen 8, 8', die dem Behälterboden 3 zugewandt sind, von dem Behälterboden 3 beabstandet. Die Wellen 8, 8' sind in Lagern 19 gelagert, die außerhalb des Lagerungsbehälters 2 an der Behälterdecke 4 angeordnet sind. Innerhalb des Lagerungsbehälters 2 sind hingegen keine Lager für die Wellen 8, 8' vorgesehen. Die Abschnitte 16, 16' der Seitenwände 5, die die Lagerungskammern 14 bilden, weisen einen Krümmungsradius, dessen Mittelpunkt auf der Wellenachse der Wellen 8, 8' liegt, auf.

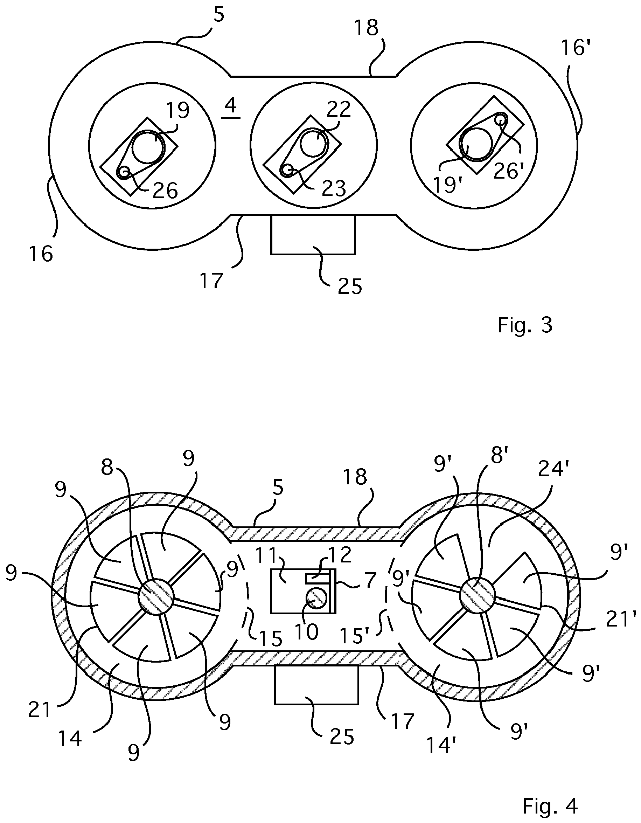

Die Wellen 8, 8' sind um ihre Längsachsen B, B' in vorgegebene Positionen drehbar. Zum Drehen der Wellen 8, 8' sind Antriebe 26, 26' vorgesehen, die ebenfalls außerhalb des Lagerungsbehälters 2 an der Behälterdecke 4 angeordnet sind. Mittels der Antriebe 26, 26' können die Wellen 8, 8' um einen vorgegebenen Winkel und ganzzahlige Vielfache davon gedreht werden. An den Wellen 8, 8' sind Halteelemente 20 ausgebildet, die es ermöglichen, Probenträger 9 an den Wellen 8, 8' zu befestigen. Die Probenträger 9 können in mehreren übereinander liegenden Ebenen 21, 21' an den Wellen 8, 8' befestigt werden (siehe insbesondere Fig. 1, Lagerungskammer 14'). Eine Ebene 21, 21' kann in gleichgroße Teile unterteilt sein, wobei in jedem Teil der Ebene 21, 21' ein Probenträger 9 an der Welle 8, 8' angeordnet sein kann. Beispielsweise kann die Ebene in sechs Teile unterteilt sein. Die Ebenen 21, 21' sind Kreisflächen, deren Kreissektoren die Teile bilden. Es ist in Fig. 4 zu erkennen, dass die dort gezeigten Ebenen 21, 21' in sechs gleichgroße Teile unterteilt sind. In jedem Teil dieser Ebene 21 kann in der Lagerungskammer 14 ein Probenträger 9 an der Welle 8 befestigt werden, also insgesamt sechs Probenträger 9. In der entsprechenden Ebene 21' der Lagerungskammer 14' sind in fünf Teilen der Ebene 21' Probenträger 9 an der Welle 8' befestigt. Ein Teil der Ebene 21', d. h. ein Lagerungssektor 24, 24', und damit ein Platz zur Aufnahme eines weiteren Probenträgers 9, ist frei.

Zwischen den beiden Lagerungseinheiten 6, 6' ist die Handhabungseinheit 7 angeordnet. Die Handhabungseinheit 7 weist eine vertikal verlaufende Welle 10 auf, die parallel und äquidistant zu den Wellen 8, 8' der Lagerungseinheiten 6, 6' in dem Lagerungsbehälter 2 angeordnet ist. Dabei liegen die Wellen 8, 8' der Lagerungseinheiten 6, 6' und die Welle 10 der Handhabungseinheit 7 in einer vertikalen Ebene. Die Welle 10 der Handhabungseinheit 7 ist durch die Behälterdecke 4 in den Lagerungsbehälter 2 geführt und erstreckt sich in Richtung des Behälterbodens 3. Dabei ist das Ende der Welle 10, das dem Behälterboden 3 zugewandt ist, von dem Behälterboden 3 beabstandet. Die Welle 10 ist in einem Lager 22 gelagert, das außerhalb des Lagerungsbehälters 2 an der Behälterdecke 4 angeordnet ist. Die Welle 10 ist um ihre Längsachse A in vorgegebene Positionen drehbar. Zum Drehen der Welle 10 kann ein Antrieb 23 vorgesehen sein, der außerhalb des Lagerungsbehälters 2 an der Behälterdecke 4 angeordnet ist.

Auf der Welle 10 der Handhabungseinheit 7 läuft ein Schlitten 11, der entlang Doppelpfeil z an der Welle 10 in Richtung der Behälterdecke 4 aufsteigen oder in Richtung des Behälterbodens 3 absteigen kann. Der Schlitten 11 weist ein Greifmittel 12 auf, das in horizontaler Richtung (Doppelpfeil x in Fig. 1) bewegt werden kann. Die Getriebe und Gestänge, mit denen die Bewegung des Schlittens 11 und des Greifmittels 12 bewirkt wird, sind in Fig. 1 nicht gezeigt.

An der Außenseite der Verbindungswand 17 kann eine Schleuse angeordnet sein, die die erfindungsgemäße Vorrichtung 1 mit einer Einbringungsvorrichtung 25 verbindet. Die Einbringungsvorrichtung 25 kann eine wärmeisolierte Kammer und einen Zugang aufweisen, über den ein Probenträger 9 in die wärmeisolierte Kammer eingebracht oder aus der wärmeisolierten Kammer entnommen werden kann. In der wärmeisolierten Kammer kann eine Transfereinrichtung angeordnet sein, die den Probenträger aus der Kammer in die Schleuse oder von der Schleuse in die Kammer führen kann.

Am Behälterboden 3 ist in dem Lagerungsbehälter 2 eine Bodenkammer (nicht gezeigt) ausgebildet, die mit flüssigem Stickstoff oder einem anderen Kältemittel gefüllt ist. Der flüssige Stickstoff oder das andere Kältemittel verdampft in dem Lagerungsbehälter 2, wodurch die Probenbehälter, die sich in dem Lagerungsbehälter befinden, gekühlt werden.

Zum Einbringen eines Probenträgers 9 in die Kryolagerungsvorrichtung 1 kann wie folgt vorgegangen werden. Zunächst wird der Probenträger 9 in die Kammer der Einbringungsvorrichtung 25 eingebracht. Mittels der Datenverarbeitungseinheit wird ein freier Lagerungssektor 24, 24' in dem Lagerungsbehälter 2 ermittelt. Der ermittelte freie Lagerungssektor 24, 24' ist der vorgegebene Lagerungssektor, d. h. der Lagerungssektor, an dem der Probenträger 9 in dem Lagerungsbehälter 2 an der Welle 8, 8' einer Lagerungseinheit 6, 6' befestigt werden soll, und gleichzeitig seine Lagerungsposition. In diesem Beispiel ist der vorgegebene Lagerungssektor ein freier Lagerungssektor 24 in der Lagerungseinheit 6. Die Transfereinrichtung führt den Probenträger 9 in die Schleuse zwischen der Einbringungsvorrichtung 25 und dem Zugang 13 zum Lagerungsbehälter 2. Dabei kommt der Probenträger 9 in der Schleuse so zu liegen, dass der äußere Rand 32 des Probenträgers 9 der Handhabungseinheit 7 zugewandt ist. Der Probenträger befindet sich nun in der Übergabeposition. Mittels der Handhabungseinheit 7 wird der Probenträger 9 durch den geöffneten Zugang 13 aus der Schleuse entnommen. Dazu wird der Schlitten 11 entlang der Welle 10 der Handhabungseinheit 7 in Höhe der Schleuse geführt. Außerdem wird der Schlitten 11 so ausgerichtet, dass das Greifmittel 12 der Schleuse und damit dem äußeren Rand 32 des Probenträgers 9 zugewandt ist. Das Greifmittel 12 wird dann horizontal bis zum äußeren Rand 32 des Probenträgers 9 bewegt. Das Greifinittel 12 greift in Ausnehmungen 35 ein, die im äußeren Rand 32 des Probenträgers 9 ausgebildet sind, wodurch das Greifinittel 12 den Probenträger 9 greift. Sobald die Verbindung zwischen dem Greifmittel 12 und dem Probenträger 9 hergestellt ist, wird das Greifmittel 12 mit dem Probenträger 9 horizontal zurück in Richtung der Welle 10 der Handhabungseinheit geführt. Anschließend kann der Zugang 13 verschlossen werden. Zwischenzeitlich wird die Lagerungseinheit 6, in der sich der vorgegebene Lagerungssektor 24 befindet, mittels ihrer Welle 8 gedreht, bis der vorgegebene Lagerungssektor der Handhabungseinheit 7 zugewandt ist. Der Schlitten 11, dessen Greifmittel 12 den Probenträger 9 hält, wird nun in Höhe der Ebene 21 geführt, in der sich der vorgegebene Lagerungssektor 24 befindet. Der Schlitten 11 wird dabei so ausgerichtet, dass der vom Greifmittel 12 gehaltene Probenträger 9 mit seinem inneren Rand 31 dem Lagerungssektor 24 zugewandt ist. Dazu wird der Schlitten um die Welle 10 der Handhabungseinheit 7 gedreht. Das Greifmittel 12 wird nun horizontal in Richtung der Welle 8 der Lagerungseinheit 6 bewegt. Sobald die Aufnahmen 34, die am inneren Rand 31 des Probenträgers 9 ausgebildet sind, in die korrespondierenden Halteelemente 20 der Welle 8 der Lagerungseinheit 6 eingreifen, so dass der Probenträger 9 an der Welle 8 befestigt ist, wird die Verbindung zwischen dem Greifmittel 12 und dem Probenträger 9 gelöst. Der Probenträger 9 befindet sich nun an seinem vorgegebenen Lagerungssektor 24 und in seiner Lagerungsposition. Sodann wird das Greifmittel 12 zurück in Richtung der Welle 10 der Handhabungseinheit 7 geführt.

Zur Entnahme eines Probenträgers 9 aus der Kryolagerungsvorrichtung 1 kann wie folgt vorgegangen werden. Mittels der Datenverarbeitungseinheit wird der Lagerungssektor 24, 24' bestimmt, an dem sich der Probenträger 9 in dem Lagerungsbehälter 2 befindet. Das ist seine Lagerungsposition. In diesem Beispiel ist dies ein Lagerungssektor 24 in der Lagerungseinheit 6. Zur Entnahme des Probenträgers 9 aus seinem Lagerungssektor 24 wird die Lagerungseinheit 6 mittels ihrer Welle 8 gedreht, bis der Lagerungssektor 24, in der sich der Probenträger 9 befindet, der Handhabungseinheit 7 zugewandt ist. Der Schlitten 11 wird in Höhe der Ebene geführt, in der sich der Lagerungssektor 24 mit dem Probenträger 9 befindet. Außerdem wird der Schlitten 11 so ausgerichtet, dass er dem Lagerungssektor 24, in dem sich der Probenträger 9 befindet, zugewandt ist. Anschließend wird das Greifmittel 12 horizontal in Richtung des Probenträgers 9 geführt, bis es in die Ausnehmungen 35, die am äußeren Rand 32 des Probenträgers 9 ausgebildet sind, eingreifen kann. Sobald die Verbindung zwischen dem Greifmittel 12 und dem Probenträger 9 hergestellt ist, wird die Verbindung zwischen dem inneren Rand 31 des Probenträgers 9 und den Halteelementen 20 an der Welle 8 gelöst. Sodann wird das Greifmittel 12 mit dem Probenträger 9 zurück in Richtung der Welle 10 der Handhabungseinheit 7 geführt. Der Schlitten 11 der Handhabungseinheit 7 wird dann entlang der Welle 10 der Handhabungseinheit 7 in Höhe der Schleuse geführt. Außerdem wird der Schlitten 11 so ausgerichtet, dass das Greifmittel 12 der Schleuse zugewandt ist. Das Greifmittel 12 wird dann mit dem Probenträger 9 horizontal durch den Zugang 13 in Richtung der Schleuse bewegt, bis sich der Probenträger 9 in der Schleuse befindet. Dort ist seine Übergabeposition. Anschließend wird die Verbindung zwischen dem Greifmittel 12 und dem äußeren Rand 32 des Probenträgers 9 gelöst und das Greifmittel zurück in Richtung der Welle 10 der Handhabungseinheit 7 geführt. Der Zugang 13 kann dann verschlossen werden. Die Transfereinrichtung entnimmt den Probenträger 9 aus der Schleuse und überführt ihn in die Kammer der Einbringungseinrichtung. Dort kann der Probenträger 9 dann beispielsweise durch einen Bediener entnommen werden.

In ähnlicher Weise kann eine Umlagerung eines Probenträgers 9 innerhalb des Lagerungsbehälters vorgenommen werden. Dazu wird der Probenträger 9 aus seinem Lagerungssektor entnommen und zu einem neuen, vorgegebenen Lagerungssektor geführt. Eine Öffnung des Zugangs 13 ist dazu nicht erforderlich.

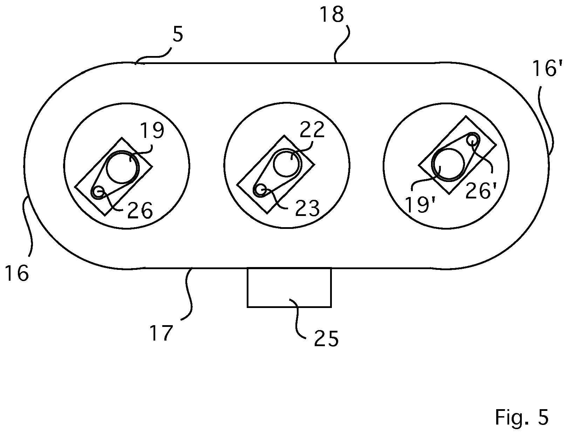

Die in Fig. 5 gezeigte zweite Ausführungsform einer erfindungsgemäßen Kryolagerungsvorrichtung 1 entspricht der in den Figuren 1 bis 4 gezeigten Ausführungsform, außer dass die Verbindungswände 17, 18 nicht nach innen versetzt sind. Vielmehr entspricht der Abstand zwischen den Verbindungswänden 17 und 18 dem Durchmesser der Lagerungskammern 14, 14'. In der ersten Ausführungsform ist der Abstand zwischen den Verbindungswänden 17 und 18 geringer als der Durchmesser der Lagerungskammern 14, 14'.

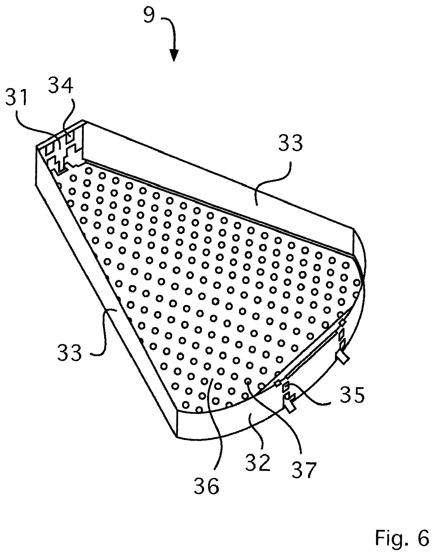

Fig. 6 zeigt einen beispielhaften Probenträger 9. Der Probenträger 9 weist einen inneren Rand 31 und einen gegenüberliegenden äußeren Rand 32 auf. Der innere Rand 31 und der äußere Rand 32 eines Probenträgers sind durch Seitenränder 33 miteinander verbunden. Der innere Rand 31 ist schmaler als der äußere Rand 32. Wird der Probenträger 9 von einer Welle 8, 8' gehalten, so erstrecken sich die Seitenränder 33 in radialer Richtung vom inneren Rand 31 zum äußeren Rand 32. Am inneren Rand 31 sind Aufnahmen 34 ausgebildet, in die Halteelemente 20, 20' der Lagerungseinheiten 6, 6' eingreifen können. Am äußeren Rand 32 sind Ausnehmungen 35 ausgebildet, in die das Greifmittel 12 der Handhabungseinheit 7 eingreifen kann. An den Unterkanten des inneren Randes 31, des äußeren Randes 32 und der Seitenränder 33 des Probenträgers 9 ist ein Boden 36 ausgebildet, der sich vom inneren Rand 31 zum äußeren 32 Rand und von dem einen Seitenrand 33 zu dem anderen Seitenrand 33 erstreckt. Der Boden weist Öffnungen 37 auf, in die korrespondierende Elemente, die an den Unterseiten der Probenbehälter ausgebildet sind, eingreifen können. Die horizontale Ausdehnung des Probenträgers 9 entspricht annähernd den Abmessungen eines Lagerungssektors 24, 24'. Der äußere Rand 32 des Probenträger 9 bildet dessen Außenkanten, wenn der Probenträger 9 an einer Welle 8, 8' der Lagerungseinheit 6, 6' befestigt ist. Die Außenkanten der Probenträger 9 können entlang eines Kreises oder Kreisbogen um die Welle 8, 8' der Lagerungseinheit 6, 6' angeordnet sein.

### Bezugszeichenliste

- 1: Kryolagerungsvorrichtung
- 2: Lagerungsbehälter
- 3: Behälterboden
- 4: Behälterdecke
- 5: Seitenwand
- 6: Lagerungseinheit
- 7: Handhabungseinheit
- 8: Welle
- 9: Probenträger
- 10: Welle
- 11: Schlitten
- 12: Greifmittel
- 13: Zugang
- 14: Lagerungskammer
- 15: Öffnung
- 16: Abschnitt der Seitenwand
- 17: Verbindungswand
- 18: Verbindungswand
- 19: Lager
- 20: Halteelement
- 21: Ebene
- 22: Lager
- 23: Antrieb
- 24: Lagerungssektor
- 25: Einbringungsvorrichtung
- 26: Antrieb

- 31: innerer Rand
- 32: äußerer Rand
- 33: Seitenrand
- 34: Aufnahme
- 35: Ausnehmung
- 36: Boden
- 37: Öffnung

## Patentansprüche

1. Kryolagerungsvorrichtung zur Lagerung von Proben, insbesondere zur Tieftemperatur-Lagerung von biologischen Proben, aufweisend einen thermisch isolierten Lagerungsbehälter (2) mit einem Behälterboden (3) und einer Behälterdecke (4), die mittels Seitenwänden (5) miteinander verbunden sind, wobei in dem Lagerungsbehälter (2) zumindest zwei Lagerungseinheiten (6, 6') ausgebildet sind, zwischen denen eine Handhabungseinheit (7) angeordnet ist, wobei
- die Lagerungseinheiten (6, 6') jeweils eine vertikal verlaufende Welle (8, 8') aufweisen, an der Probenträger (9) zur Aufnahme von Probenbehältern lösbar befestigt werden können;
- die Handhabungseinheit (7) eine vertikal verlaufende Welle (10) aufweist, an der ein Schlitten (11) läuft, wobei der Schlitten (11) in vertikaler Richtung entlang der Welle (10) beweglich ist, um eine Drehachse (A) drehbar ist, die auf der Welle (10) der Handhabungseinheit liegt, und ein in horizontaler Richtung bewegliches Greifmittel (12) aufweist, das in lösbaren Eingriff mit einem Probenträger (9) gebracht werden kann;
- zumindest in einer Seitenwand (5) des Lagerungsbehälters (2) ein verschließbarer Zugang (13) ausgebildet ist, zu dem der Probenträger (9) mittels des Schlittens (11) und des Greifmittels (12) führbar ist.

2. Kryolagerungsvorrichtung nach Anspruch 1, **dadurch gekennzeichnet, dass** die Wellen (8, 8') der Lagerungseinheiten (6, 6') und die Welle (10) der Handhabungseinheit (7) durch die Behälterdecke (4) geführt sind, wobei jede der Wellen (8, 8', 10) in einer Lagereinheit (19, 19', 22) außerhalb des Lagerungsbehälters (2) gelagert ist.

3. Kryolagerungsvorrichtung nach Anspruch 2, **dadurch gekennzeichnet, dass** sich die Wellen (8, 8', 10) innerhalb des Lagerungsbehälters (2) in Richtung des Behälterbodens (3) erstrecken, ohne in einer Lagerungseinheit am Behälterboden (3) gelagert zu sein.

4. Kryolagerungsvorrichtung nach Anspruch 2 oder Anspruch 3, **dadurch gekennzeichnet, dass** außerhalb des Lagerungsbehälters (2) Antriebe (26, 26') für die Wellen (8, 8') der Lagerungseinheiten (6, 6') und/oder ein Antrieb (23) für die Welle (10) der Handhabungseinheit (7) angeordnet sind.

5. Kryolagerungsvorrichtung nach einem der vorstehenden Ansprüche, **dadurch gekennzeichnet, dass** die Probenträger (9) in mehreren, in vertikaler Richtung übereinander liegenden Ebenen (21, 21') an einer Welle (8, 8') einer Lagerungseinheit (6, 6') befestigbar sind.

6. Kryolagerungsvorrichtung nach Anspruch 5, **dadurch gekennzeichnet, dass** in einer Ebene (21, 21') mehrere Probenträger (9) anordenbar sind, wobei die Außenkanten (32) der Probenträger (9), die in einer Ebene (21, 21') angeordnet sind, entlang eines Kreises oder Kreisbogens um die Welle (8, 8') der Lagerungseinheit (6, 6') angeordnet sind.

7. Kryolagerungsvorrichtung nach einem der vorstehenden Ansprüche, **dadurch gekennzeichnet, dass** innerhalb des Lagerungsbehälters (2) am Behälterboden (3) eine Bodenkammer zur Aufnahme von flüssigem Stickstoff ausgebildet ist.

8. Kryolagerungsvorrichtung nach Anspruch 7, **dadurch gekennzeichnet, dass** an der Behälterdecke (4) zumindest eine Deckenkammer zur Aufnahme von flüssigem Stickstoff ausgebildet ist, wobei zumindest eine der Deckenkammern über eine Leitung mit der Bodenkammer verbunden ist.

9. Kryolagerungsvorrichtung nach einem der vorstehenden Ansprüche, **dadurch gekennzeichnet, dass** der Lagerungsbehälter (2) Lagerungskammern (14, 14') aufweist, in denen jeweils eine Lagerungseinheit (6, 6') angeordnet ist, wobei die Lagerungskammern (14, 14') in Richtung der Handhabungseinheit (7) geöffnet sind.

10. Kryolagerungsvorrichtung nach Anspruch 9, **dadurch gekennzeichnet, dass** der Mantel (16, 16') einer Lagerungskammer (14, 14') von einer Seitenwand (5) des Lagerungsbehälters (2) gebildet ist und der Behälterboden (3) die Lagerungskammer (14, 14') nach unten begrenzt, wobei der Behälterboden (3) im Bereich der Lagerungskammer (14, 14') nach außen gewölbt ist.

11. Kryolagerungsvorrichtung nach einem der vorstehenden Ansprüche, **dadurch gekennzeichnet, dass** die Seitenwand des Lagerungsbehälters (2), in der der verschließbare Zugang (13) ausgebildet ist, eine Seitenwand (17) des Lagerungsbehälters (2) ist, die zwischen den Seitenwänden (16, 16') zweier benachbarter Lagerungskammern (14, 14') ausgebildet ist.

12. Kryolagerungsvorrichtung nach einem der vorstehenden Ansprüche, **dadurch gekennzeichnet, dass** der verschließbare Zugang (13) eine Schleuse ist, über die die Vorrichtung (1) mit einer Einbringungsvorrichtung (25) verbunden ist, die einen Arbeitsraum aufweist, über den ein Probenträger (9) von der Einbringungsvorrichtung (25) an die Handhabungseinheit (7) der Kryolagerungsvorrichtung (1) oder von der Handhabungseinheit (7) der Kryolagerungsvorrichtung (1) an die Einbringungsvorrichtung (25) überführbar ist.

13. Kryolagerungsvorrichtung nach einem der vorstehenden Ansprüche, **dadurch gekennzeichnet, dass** an den Wellen (8, 8') der Lagerungseinheiten (6, 6') Halteelemente (20, 20') ausgebildet sind, die in korrespondierende Aufnahmen (34) eingreifen können, die an den Probenträgern (9) ausgebildet sind.

14. Verfahren zur Lagerung von Proben, insbesondere zur Tieftemperatur-Lagerung von biologischen Proben, mittels einer Kryolagerungsvorrichtung (1) nach einem der Ansprüche 1 bis 13, **dadurch gekennzeichnet, dass** ein Probenträger (9) mittels der Handhabungseinheit (7) zwischen einer Übergabeposition und einer Lagerungsposition bewegt wird, wobei
- sich der Probenträger (9) in der Übergabeposition an dem verschließbaren Zugang (13), der in einer Seitenwand (17) des Lagerungsbehälters (2) ausgebildet ist, befindet und
- sich der Probenträger (9) in der Lagerungsposition in einer vorgegebenen Lagerungseinheit (6, 6') an einer vorgegebenen Position (24, 24') an einer Welle (8, 8') der Lagerungseinheit (6, 6') befindet.

15. Verfahren nach Anspruch 14, **dadurch gekennzeichnet, dass** zur Überführung des Probenträgers (9) von der Übergabeposition zu einer vorgegebenen Lagerungsposition oder von seiner Lagerungsposition zu der Übergabeposition der Probenträger (9) mittels des Greifmittels (12) in horizontaler Richtung und mittels des Schlittens (11) in vertikaler Richtung bewegt wird.
